(19) Europäisches Patentamt
European Patent Office
Office européen des brevets

(11) **EP 3 300 694 A1**

(12) **EUROPEAN PATENT APPLICATION**

(43) Date of publication:
**04.04.2018 Bulletin 2018/14**

(51) Int Cl.:
***A61F 2/16*** (2006.01)      ***G02C 7/02*** (2006.01)

(21) Application number: **16191795.0**

(22) Date of filing: **30.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **SAV-IOL SA**
**2000 Neuchatel (CH)**

(72) Inventor: **PASCARELLA, Alexandre**
**2013 Colombier (CH)**

(74) Representative: **Leman Consulting S.A.**
**Chemin de Précossy 31**
**1260 Nyon (CH)**

(54) **METHOD FOR DETERMINING GEOMETRIC PARAMETERS OF AN OPHTHALMIC LENS AND OPHTHALMIC LENS OBTAINED BY THE IMPLEMENTATION OF THIS METHOD**

(57)    The present invention concerns a method for determining geometrical parameters of an ophthalmic lens intended to be implanted in a patient's eye. This method comprises the following steps:
• measuring biometric data specific to the patient;
• choosing characteristics for the vision of the patient after correction;
• choosing non biometric parameters of the lens;
• calculating a light distribution specification from the biometric data, from the non biometric parameters and from said vision characteristics chosen, wherein the light distribution specification defines, among others, which portions of the available light are allocated to different distances of vision;
• calculating geometrical parameters for the lens in such a way that the set formed by the ophthalmic lens and the eye of the patient generates a light distribution specification corresponding to the light distribution specification calculated.

The invention also concerns an ophthalmic lens comprising a base correcting optical element and at least one phase distribution element. The geometrical parameters of said base correcting optical element are determined from the result of measuring biometric data specific to the patient and of choosing non biometric parameters of the lens. The geometrical parameters of said at least one phase distribution element are determined from the result of calculating a light distribution specification depending on visual characteristics chosen for a patient after correction.

Fig. 1

Printed by Jouve, 75001 PARIS (FR)

EP 3 300 694 A1

## Description

## TECHNICAL FIELD

The present invention concerns a method for determining geometric parameters of an ophthalmic lens intended for a patient. It further concerns an ophthalmic lens obtained by this method.

The ophthalmic lens can be any intraocular lens implanted in a patient's eye such as pseudo-phakic intraocular lens (IOL) or a phakic IOL for anterior or posterior chambers.

## BACKGROUND ART

In the current state of the art, when the vision of a patient must receive an optical correction due to a vision disorder, especially when this correction is made by an intraocular lens, the optical characteristics of the eye of the patient are measured. In particular, the type of correction is determined as well as the extent of the correction. When the type and extent of the correction have been determined, the lens which is most adapted to the optical characteristics of the patient is chosen among a range of available intraocular lenses. This lens is then generally implanted in the patient's eye, for example to replace the crystalline lens of the eye in the case of cataract surgery. In general, the available lenses exist in a predefined range of powers, for example every 0.5 diopter, to fit with the patient eye biometry, and often to correct myopia, hypermetropia and astigmatism.

A problem with this kind of lens is that it only takes into account the optical characteristics of the patient's eye. Each patient with the same optical characteristics will receive the same lens. In practice, this is not satisfactory because the needs and expectations of different patients may vary. In particular, depending on the main activities of each patient, the lenses implanted onto the patient should be adapted.

## DISCLOSURE OF THE INVENTION

An object of the invention is obtained by a method for determining geometrical parameters of an ophthalmic lens intended for a patient, this method comprising the following steps:

- measuring biometric data specific to the patient;
- choosing characteristics for the vision of the patient after correction;
- choosing non biometric parameters of the lens;
- calculating a light distribution specification from the biometric data, from the non biometric parameters and from said vision characteristics chosen;
- calculating geometrical parameters for the lens in such a way that the set formed by the ophthalmic lens and the eye of the patient generates a light distribution specification corresponding to the light distribution specification calculated.

Another object of the invention is also obtained by an ophthalmic lens comprising a base correcting optical element and at least one phase distribution element, geometrical parameters of said base correcting optical element being determined from the result of measuring biometric data specific to the patient and of choosing non biometric parameters of the lens, geometrical parameters of said at least one phase distribution element being determined from the result of calculating a light distribution specification depending on visual characteristics chosen for a patient after correction.

According to this invention, the correction to be made for a specific patient is first measured in a conventional way, to determine the type and extent of the correction which is necessary to correct the patient's vision problems. The extent of the correction determines the power of the lens. The patient can then define his/her needs in terms of near vision, intermediate vision and distant vision. This information is then used, in combination with biometric information specific to this patient, and is transformed to define geometrical parameters of a lens specific to this user and corresponding to the patient's expectations. A specific lens can then be made based on these geometrical parameters.

Unlike the lenses of the prior art, in which only the power is selected, the lens of the present invention can offer the patient a vision that really corresponds to his needs and expectations.

The present invention is also advantageous in that it can be carried out on a lens having an extended depth of focus while allowing for the distribution of the light in an individual way for each patient.

The present invention concerns on one hand an ophthalmic lens which can be implanted in an eye of a patient to correct eyesight disorder or eye pathology. The invention can also be used in case of cataract surgery, in replacement of the crystalline lens of the patient. On the other hand, the invention concerns a method for determining geometric parameters of said ophthalmic lens in order to enable the realization of such an ophthalmic lens.

An ophthalmic lens of this invention can be considered as being composed of at least two elements. One of these elements is a base correcting optical element and at least one other element is a phase distribution element.

According to the invention, the ophthalmic lens is seen as an assembly of two elements. The base corrective optical element is essentially determined so as to take into consideration the type of correction to the patient and the extent of the correction. The phase distribution element or elements, is determined to take into account parameters related to the type of vision that the patient wishes to have once corrected.

In the method of the invention, the shape of the base correcting optical element and the shape of the phase distribution element are calculated separately. More specifically, the method comprises a first step in which the needs of the patient are processed, together with biometric information specific to that patient. The processing results in a light distribution specification. The biometric data are used to define the shape and other characteristics of the base correcting optical element. The light distribution specification is then used to define the geometrical parameters of a phase distribution element, the shape of the phase distribution element being determined by calculation for generating a light distribution corresponding to the light distribution specification previously defined.

## BRIEF DESCRIPTION OF DRAWINGS

The present invention and its advantages will be better understood with reference to the enclosed drawings and to a detailed description of specific embodiments in which:

- Figure 1 is a bloc diagram illustrating the method of the invention;

- Figure 2 illustrates a light distribution corresponding to a patient's expectations;

- Figure 3 is a schematic cross section view of a first embodiment of a lens according to the present invention;

- Figure 4 illustrates a light intensity as a function of the power of the lens of Figure 3;

- Figure 5 is a schematic cross section view of a second embodiment of a lens according to the present invention;

- Figure 6 illustrates a light intensity in function of the power of the lens of Figure 5;

- Figure 7a illustrates a light intensity as a function of the power of a lens having a first extended depth of focus (E-DOF); and

- Figure 7b is similar to Figure 7a, for another extended depth of focus.

## MODE FOR CARRYING OUT THE INVENTION

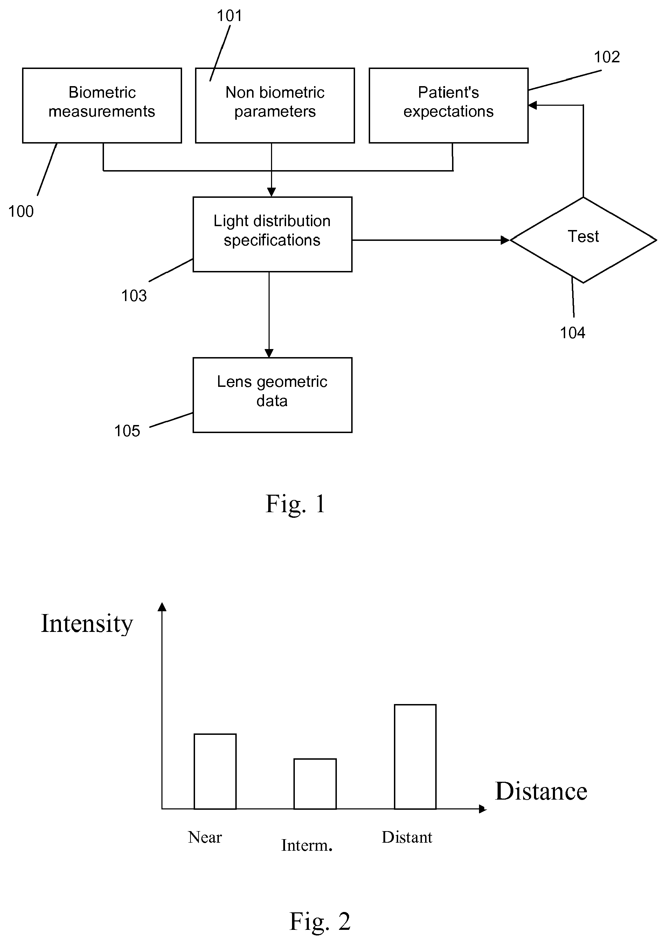

With reference to Figure 1, the method of the invention is intended to calculate geometric parameters used to define the geometrical shape of an ophthalmic lens and thus, to enable manufacturing of this lens.
The method of the invention operates as follows. In a first step, referred to as 100 on Fig. 1, various features of the patient's eye are determined. These features are among others, biometric information and in particular the type of correction required and the amount or extent of the correction. The type of correction is defined by the type of visual disturbance that the ophthalmic lens of the present invention has to correct. Visual disturbances such a lens is able to correct include myopia, hypermetropia and astigmatism (second-order aberrations). The extent of the correction corresponds to a distant-vision correction. The extent of the correction and the type of correction are generally conventionally used nowadays while correcting a patient's vision.
Other biometric parameters may also be measured and in particular parameters related to dimensions. These measurements are intended to ensure that the dimensions and shape of the lens are optimal after implantation in a patient's eye.
Alternatively, other characteristics of the eye can be measured. These are for example the existence of aberrations such as spherical aberration, chromatic aberration, or more generally aberrations having an order higher than two. Such aberrations can for example be in the periphery of the cornea and are responsible for the distortion of vision when the pupil dilates. Some visual difficulties they can cause comprise halos and glares in night vision. The profile of the higher order aberrations is specific to each eye. These aberrations can be measured and corrected individually by means of the lens of the invention.
Other "non biometric" parameters of the lens are chosen in a step 101. These parameters are chosen among at least:

- the type of lens
- the material of the lens
- the depth of focus
- the extended depth of focus
- the haptic diameter
- the haptic type
- the toricity
- the add value

Other parameters could also be selected, depending on the implementation.
A next step 102 of the method of the invention comprises determining the visual characteristics desired by the patient after correction. These visual characteristics depend in particular on the needs and activities of the patient. For example, for a person who does not drive and reads a lot, near vision will be promoted at the expense of intermediate and distant vision. For a person using a computer frequently, vision at an intermediate distance will be preferred. Distant vision will be promoted in the case of a person driving regularly, for example.
According to an embodiment of the method of the invention, it is possible to define several zones, for example a first zone called near vision zone corresponding to a range typically between 20 and 50 cm from the patient. An intermediate vision zone is defined as covering a range between 50 cm and 1.50 m, and a third zone, called distant vision zone corresponds for example to a viewing distance extending beyond 1.50 m. It should be noted that a different number of zones may be defined and/or different distances may be chosen. It is also possible not to separate the viewing distance in discrete zones, but rather to use a continuous distance.

During this step 102, the user defines his/her needs or requirements related to vision after implantation of the ophthalmic lens. This step is also illustrated by Figure 2. In this figure, three rectangles are shown. One represents the light intensity expected by the patient for near vision; the middle rectangle represents the expected light intensity for intermediate vision and the rectangle on the right illustrates the light intensity expected for distant vision. The sum of the height of these rectangles may represent 100% of the available light intensity. As mentioned above, the number of rectangles is not limited to three. It is possible to use only two zones or more than three. It is also possible to replace the discrete rectangles by a continuous zone or curve.

In a subsequent step 103 of the method, the biometric measurements obtained after step 100 and the non biometric parameters obtained in step 101, as well as the requirements defined by the patient during step 102 are converted into a light distribution specification. This light distribution specification defines among others, which portions of the available light are allocated to different distances of vision.

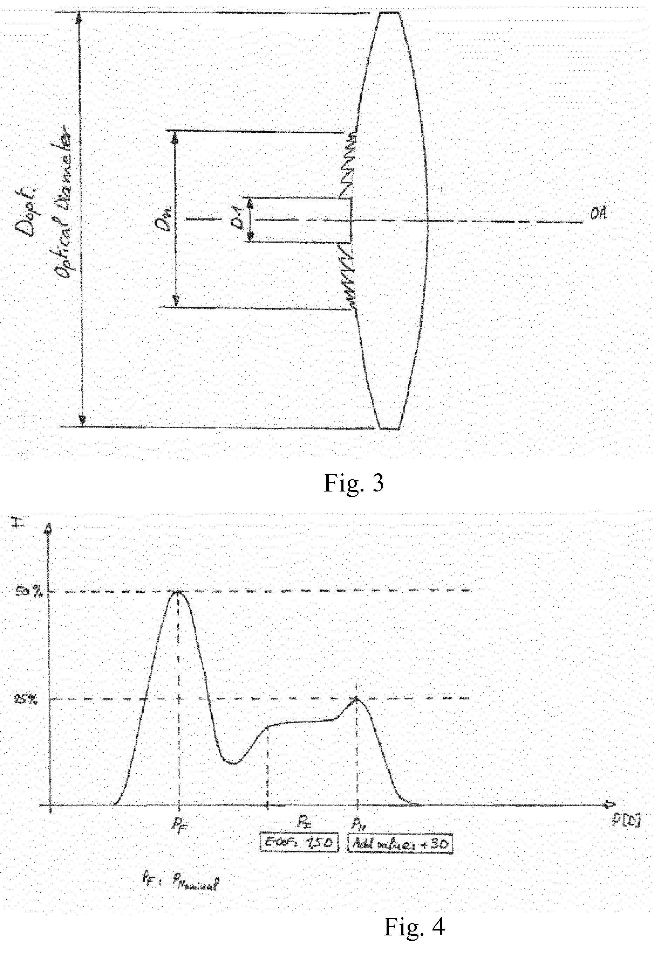

Light distribution specifications are at least partially schematically illustrated by Fig. 4, 6, 7a and 7b. More specifically, the curves of Figures 4, 6, 7a and 7b illustrate a light intensity as a function of an optical power of the lens and are obtained by processing among others, the information illustrated by Fig. 2.

An image at infinity focuses on the patient's retina for a lens having a given power (for example 22 diopters). An image close to the patient focuses on the retina for a lens having another power (for example 25 diopters). For a given power, and thus, for a given distance "object side", only a part of the intensity will focus on the retina and will thus be useful "image side". Figures 4, 6, 7a and 7b represent the intensity depending on the power of the lens.

The power required for a given distance depends on the patient and in particular, whether this patient is emmetropic or not. This information is determined among others during the step 100 of the method of the invention.

It should be noted that there is a one-to-one relationship between the power of the lens and the distance. Thus, it is possible to draw or calculate a curve representing the intensity as a function of a distance from a curve representing the intensity as a function of a power. However, as the power is calculated as an inverse of a focal distance, the left and right parts of the curves will be inverted. This means that the left part of the curves of Figures 4, 6, 7a and 7b (low power) corresponds to distant vision and thus, to the right part of Fig. 2. The right part of the curves of Figures 4, 6, 7a and 7b (high power) corresponds to near vision and to the left part of Figure 2.

In Figures 4, 6, 7a and 7b, the vertical axis represents the light intensity in percent. The total area of the curve represents 100%.

In Fig. 4, a first peak is placed at a first power $P_F$ corresponding to far vision distance. In the example illustrated, the intensity at this first power corresponds to 50% of all the available light intensity. Another local peak is set at a second power $P_N$ corresponding to near vision. Finally, the rest of the available intensity is distributed in particular in the intermediate vision so that 100% of the intensity is distributed. The curve illustrated by Figure 4, showing the intensities as a function of power, can correspond to the histograms of Figure 2.

Figure 3 illustrates a lens that can be used to produce an intensity distribution as represented on Figure 4. This lens is formed of a base optical element represented as a convex lens, and of a phase distribution element. This phase distribution element is represented as an annular structure realized on side of the lens. The shape and other characteristics of the base optical element define the distribution of the light for near, intermediate and distant vision.

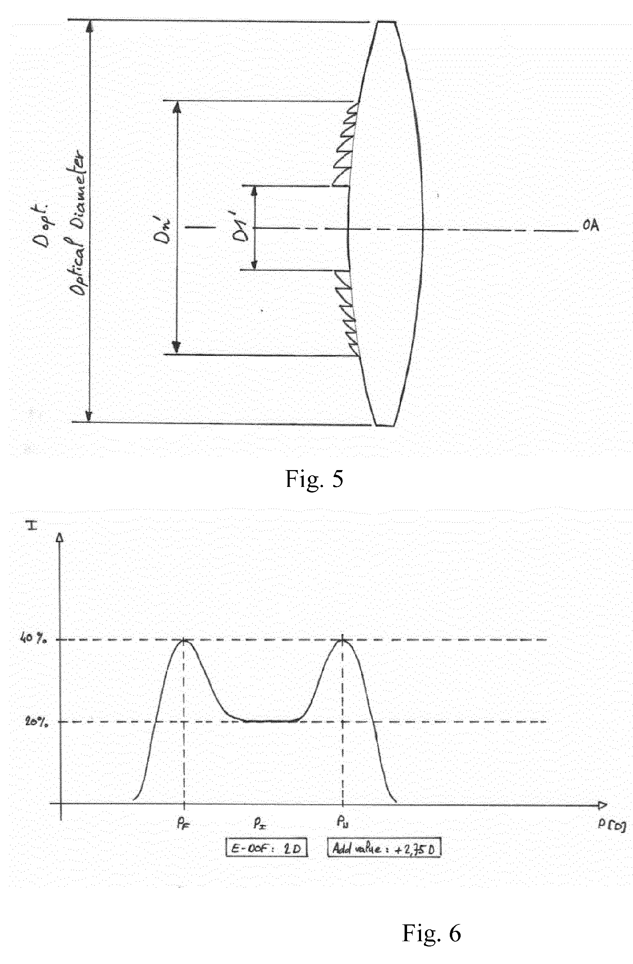

In Fig. 6, the light distribution curve shows two equal peaks at a first power $P_F$ and a second power $P_N$ corresponding respectively to far vision and near vision. These peaks are linked by a zone receiving less intensity and corresponding to intermediate vision.

Fig. 5 is similar to Figure 3 and illustrates a lens producing an intensity distribution of Figure 6. Modifications of parameters of the lens such as the diameters of the different parts of the lens, of the shape, height and width of the diffractive structure influences the light intensity distribution.

As mentioned previously, several parameters are used to define light distribution specifications. Among them, two are detailed below. A first one is the add value. This add value indicates how much power is added to the lens to create a near vision zone on the lens. In terms of intensity curves as represented by figures 4, 6, 7a and 7b, modifying the add value modifies the position and the height of the right part of the curve.

Another parameter is the extended depth of focus. This parameter defines the extension of the area in which the image is focused.

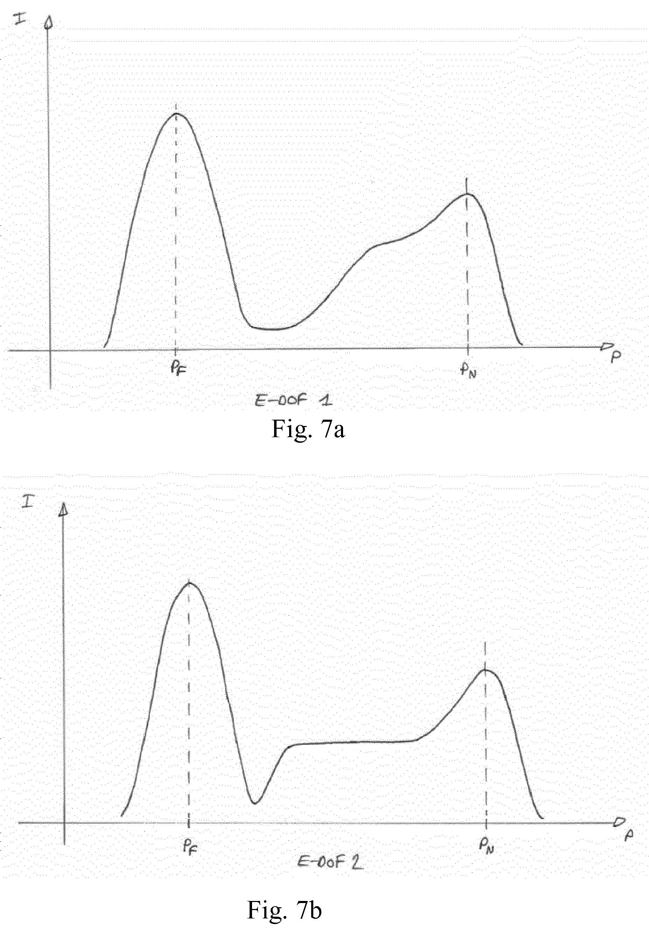

Figure 7a shows a light intensity curve with a first extended depth of focus (E-DOF = 1). Figure 7b illustrates a light intensity curve with a second extended depth of focus (E-DOF = 2), the other parameters of the lens generating these curves being equivalent. As it can be seen by comparing these curves, the peaks corresponding to distant and near vision are in the same positions and have similar intensities for both curves. For the intermediate vision, the intensity is more constant for a lens having a greater extended depth of focus.

It is possible to define for each zone corresponding to a rectangle in Figure 2, a minimum threshold for the vision. In other words, even if the patient sets the value of at least one of the zones corresponding to near, intermediate or distant vision to zero, a residual intensity will be considered. Thus, the user will always have a minimal vision whatever the distance considered.

As it can be seen on these curves, between the minimal and maximal values of power with which light intensity is received, there is no zone receiving no light intensity. This ensures a more conformable vision to a patient and avoids him/her being "blind" for a given distance of vision without additional visual correction provided for example by glasses.

According to an optional but interesting embodiment, it is possible to simulate the vision that will be obtained by the patient with the light distribution specifications as defined in step 102 and to allow the patient to test this simulation. This simulation allows the patient to check whether the light distribution parameters have been properly selected and if they generate a vision corresponding to the expectations of the patient. If such correspondence is not met, these parameters can be modified and tested again until the desired vision matches the simulated vision. This test is illustrated under the reference 103 in Fig. 1.

Once the distribution of the light established with or without correction by a simulation, this distribution is used to calculate geometric parameters of the lens in such a way that the assembly formed by the ophthalmic lens and the patient eye leads to the light distribution specification as defined in the previous step 102.

A base refractive optical element is defined to get the desired nominal dioptric power of the ophthalmic lens. Additionally, one or several diffractive structures can be used to distribute the light required for other powers. Pseudo non diffracting beams (PNDB) can be used to extend the depth of focus, participating in the light distribution.

The ophthalmic lens comprises an anterior and a posterior surface. Both of these surfaces can be used to distribute the light along the optical axis or to correct visual aberrations by means of multiple optical elements in several zones or surfaces.

The base refractive optical element contributes to set the nominal power from its surface curvatures combined with the refractive index of the lens material. The geometrical profiles of the anterior and/or posterior surfaces can be convex, concave or flat.

Additional optical elements can be used on defined zones of the lens to contribute to a pseudo-accommodation by distributing light intensity along the optical axis on the useful region for a natural vision (visual acuity of a healthy eye with accommodation, emmetropic situation). The amount of energy transmitted by the lens for each distance (far-intermediate-near vision) is a function of the surface of each exposed optical element and their intrinsic properties. The conversion of a desired light distribution specification into optical surfaces is therefore dependent on these two aspects. Moreover, the exposed surface of the lens increasing as a square function of the pupil radius, the intensity distribution according to the given model may be set for a standard average pupil (e.g. 3mm) but may also be designed and optimized as a function of the pupillary diameter by lowering any strong visual acuity variation for a rapid diameter change.

From a defined light distribution specification (for example 50% of intensity for far vision, 25% for intermediate vision and 25% for near vision), the surface of each optical element area, combined with its optical properties, is calculated in proportion. The surface allocated for a refractive element can result for example in a 100% intensity ratio distributed for the far vision. A surface allocated for a diffractive optical element such as a diffraction grating (kinoform, pattern of concentric rings) can be used combined with an apodisation model to fine tune the light distribution function of the eye pupil aperture between the far vision and another chosen focusing distance (or more, depending on the diffraction order) by splitting the intensity. Light energy can be then distributed with a ratio that is a function of the pupil aperture to fit the initial desired model. The splitting ratio is mainly driven by the height of the diffractive structures while the added power by their width. Each of these different optical elements has also more or less intrinsic loss of energy that is taken into consideration to reach the desired ratios.

Pseudo-accommodation can be reached also by extending the depth of focus of the lens. A pseudo-non diffracting beam (PNDB) can be created with a phase mask that will transform the incident wave front into a beam with a constant intensity while maintaining a satisfactory resolution along a defined distance on the optical axis.

The created beam is non diverging along a finite propagation distance. The positioning of the PNDB along the optical axis allows distributing light intensity along an extended depth of focus (E-DOF) for an expected visual acuity. Combined with other optical elements of the ophthalmic lens, this type of beam can be used to extend the visual acuity for example from the near vision to the distant vision or even to create a junction between the far and near vision. The beam can be calculated and shifted along the optical axis to provide more or less of a transition between the different vision distances and with more or less overlap between these visions, the beam length (E-DOF) can vary as well.

Definition of the PNDB to extend the depth of focus can be done for example through the parameterization of a radial harmonic function. The radial harmonic function is chosen to be a phase only filter. Based on the expected depth of focus, the interval where the beam has a near constant intensity on the optical axis is defined and combined with the pupil aperture. This sets the parameters of the harmonic function. This interval can be also shifted along the optical axis to distribute the intensity at the required distance.

Doing so, the near constant intensity along a finite distance can be positioned to allocate this intensity in accordance with the desired model. For example, a pseudo-phakic intraocular lens (IOL) with a nominal power P for distant vision can be combined with an add value of +3D for near vision provided either by diffractive concentric circular structures or with a PNDB or a combination of both. A PNDB can be used to cover the intermediate vision range with an extended depth of focus of some diopters (e.g. 1, 1.5, 2, 2.5,...). The through focus response of the optical system must be considered to provide a sufficient resolution for each vision. Any other combination is feasible to reach the expected target and for any intraocular lens type.

In the examples of Figures 3 and 5, a central zone of surface S (e.g. 1 mm$^2$) on the optic can be used to allocate intensity for the near and intermediate vision by extending the depth of focus. A second zone with an order of magnitude of 10*S can be used to allocate intensity both for the near and far vision by splitting the intensity. A third zone with an order of magnitude of 20*S can be used to allocate intensity only for the far vision. The intensity distribution resulting from these surfaces is then dependent of the pupillary aperture. The through focus response of the optical system must be considered to provide a sufficient resolution for each vision. Any other combination is feasible to reach the expected target and for any intraocular lens type (without limitation regarding the number of zones, the surfaces areas and the use of the anterior or posterior optical surface of the lens).

The phase data is converted into geometrical data by considering the wavelength and the refractive index of the lens material by the relation:

$$t(r) = \frac{\lambda \Phi(r)}{2\pi(n-1)}$$

The surface of each optical element contributes to the required ratio in terms of light distribution or visual acuity.

Different areas can also combine their effects resulting in some overlaps when focusing the light to defined focal lengths to reach the desired intensities at these distances.

To get the desired light distribution with the expected intensity ratios, the optic includes any (but at least one) optical element that will, depending on its exposed surface and optical characteristics, focus the light collected on this area to a defined distance on the optical axis.

Any other optical aberration correction element can be included as a custom-made option.

The method and the lens of the invention enables providing ophthalmic lenses that do not only correct patient's vision diseases, but that are also adapted to the patient's requirements.

**Claims**

1. Method for determining geometrical parameters of an ophthalmic lens intended for a patient, this method comprising the following steps:

   • measuring biometric data specific to the patient;
   • choosing characteristics for the vision of the patient after correction;
   • choosing non biometric parameters of the lens;
   • calculating a light distribution specification from the biometric data, from the non biometric parameters and from said vision characteristics chosen;
   • calculating geometrical parameters for the lens in such a way that the set formed by the ophthalmic lens and the eye of the patient generates a light distribution specification corresponding to the light distribution specification calculated.

2. Method for determining geometrical parameters of an ophthalmic lens according to claim 1, wherein the step of choosing vision characteristics for the patient after correction comprises a step of selecting an amount of light intensity as a function of a distance of vision.

3. Method for determining geometrical parameters of an ophthalmic lens according to claim 1, wherein the step of choosing vision characteristics for the patient after correction comprises selecting aberrations to be corrected.

4. Method for determining geometrical parameters of an ophthalmic lens according to claim 2 or 3, wherein said method further comprises a step of simulating the characteristics of the vision of the patient after correction and a step of modifying the characteristics chosen by the patient in the case that the characteristics simulated do not correspond to the characteristics chosen for the patient after correction.

5. Method for determining geometrical parameters of an ophthalmic lens according to claim 1, wherein the step of choosing non biometric parameters of the lens comprises choosing a value of at least one of the following parameters among :

   • a type of lens;
   • a material of the lens;
   • a depth of focus;
   • an extended depth of focus;
   • a haptic diameter;
   • a haptic type;
   • a toricity;
   • an add value.

**6.** Method for determining geometrical parameters of an ophthalmic lens according to claim 1, wherein the result of the step of measuring biometric data specific to the patient and of choosing non biometric parameters of the lens is used to calculate the geometrical parameters of a base correcting optical element and wherein the result of the step of calculating a light distribution specification is used to calculate the geometrical parameters of at least one phase distribution element.

**7.** Method for determining geometrical parameters of an ophthalmic lens according to claim 1, wherein the light distribution specification comprises phase data.

**8.** Method for determining geometrical parameters of an ophthalmic lens according to claim 7, wherein said phase data is converted into geometrical data by using the relation :

$$t(r) = \frac{\lambda \Phi(\mathrm{r})}{2\pi(n-1)}$$

where $\lambda$ is the wavelength; $\Phi$ is the phase and $n$ is the refractive index of the lens material.

**9.** Ophthalmic lens comprising a base correcting optical element and at least one phase distribution element, geometrical parameters of said base correcting optical element being determined from the result of measuring biometric data specific to the patient and of choosing non biometric parameters of the lens, geometrical parameters of said at least one phase distribution element being determined from the result of calculating a light distribution specification depending on visual characteristics chosen for a patient after correction.

**10.** Ophthalmic lens according to claim 9, wherein said lens comprises at least two different optical areas, each area having a surface calculated to match the light distribution specification calculated according to the method of claim 1.

101

100

| Biometric measurements | Non biometric parameters | Patient's expectations |
|---|---|---|

102

Light distribution specifications

103

Test

104

Lens geometric data

105

Fig. 1

Intensity

Near     Interm.     Distant

Distance

Fig. 2

Fig. 3

Fig. 4

Fig. 5

Fig. 6

Fig. 7a

Fig. 7b

Europäisches
Patentamt

European
Patent Office

Office européen
des brevets

# EUROPEAN SEARCH REPORT

Application Number

EP 16 19 1795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/074742 A1 (AMO GRONINGEN BV [US]; WEEBER HENDRIK A [NL]) 7 June 2012 (2012-06-07) | 1-10 | INV. A61F2/16 G02C7/02 |
| Y | * paragraphs [0013], [0019], [0031], [0033], [0061] - [0063], [0065], [0069], [0078]; figure 5 * | 4 | |
| X | EP 1 891 912 A1 (ALCON MFG LTD [US]) 27 February 2008 (2008-02-27) | 1-3,5-10 | |
| Y | * figures 1, 2A * * paragraphs [0002], [0004], [0006] - [0010], [0015], [0036] - [0039], [0047] * | 4 | |
| X | WO 2009/076670 A1 (ADVANCED MEDICAL OPTICS INC [US]; VAN DER MOOREN MARIE H [NL]; PIERS P) 18 June 2009 (2009-06-18) | 9,10 | |
| A | * paragraphs [0018], [0019], [0030]; figures 1-3 * | 1-8 | |
| X | US 2010/321635 A1 (APTER ROBERT [CH] ET AL) 23 December 2010 (2010-12-23) | 9,10 | TECHNICAL FIELDS SEARCHED (IPC) |
| A | * paragraphs [0003], [0110], [0111]; figure 3 * | 1-8 | A61F G02C |

The present search report has been drawn up for all claims

| Place of search | Date of completion of the search | Examiner |
|---|---|---|
| Munich | 24 March 2017 | Vazquez Martinez, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 19 1795

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2017

| Patent document cited in search report | | Publication date | Patent family member(s) | | Publication date |
|---|---|---|---|---|---|
| WO 2012074742 | A1 | 07-06-2012 | AU | 2011337014 A1 | 20-06-2013 |
| | | | CA | 2819451 A1 | 07-06-2012 |
| | | | EP | 2646871 A1 | 09-10-2013 |
| | | | US | 2012296422 A1 | 22-11-2012 |
| | | | US | 2014081395 A1 | 20-03-2014 |
| | | | WO | 2012074742 A1 | 07-06-2012 |
| EP 1891912 | A1 | 27-02-2008 | AR | 062487 A1 | 12-11-2008 |
| | | | AU | 2007209782 A1 | 13-03-2008 |
| | | | BR | PI0703530 A | 22-04-2008 |
| | | | CA | 2597376 A1 | 23-02-2008 |
| | | | CN | 101129278 A | 27-02-2008 |
| | | | EP | 1891912 A1 | 27-02-2008 |
| | | | JP | 2008049167 A | 06-03-2008 |
| | | | KR | 20080018146 A | 27-02-2008 |
| | | | TW | 200819117 A | 01-05-2008 |
| | | | US | 2007171362 A1 | 26-07-2007 |
| | | | US | 2011022170 A1 | 27-01-2011 |
| WO 2009076670 | A1 | 18-06-2009 | AU | 2008334939 A1 | 18-06-2009 |
| | | | CA | 2709105 A1 | 18-06-2009 |
| | | | EP | 2229091 A1 | 22-09-2010 |
| | | | WO | 2009076670 A1 | 18-06-2009 |
| US 2010321635 | A1 | 23-12-2010 | EP | 2243053 A2 | 27-10-2010 |
| | | | US | 2010321635 A1 | 23-12-2010 |
| | | | WO | 2009115932 A2 | 24-09-2009 |

EPO FORM P0459

For more details about this annex : see Official Journal of the European Patent Office, No. 12/82